Europäisches Patentamt
European Patent Office
Office européen des brevets

(19) (11) Publication number: 0018155 A2

# (12) EUROPEAN PATENT APPLICATION

(21) Application number: 80301118.8

(22) Date of filing: 08.04.80

(51) Int. Cl.³: C 07 F 9/65, A 61 K 31/675

(30) Priority: 09.04.79 DK 1455/79

(43) Date of publication of application: 29.10.80 Bulletin 80/22

(84) Designated Contracting States: CH DE FR GB LI SE

(71) Applicant: NOVO INDUSTRI A/S, Novo Allé, DK-2880 Bagsvaerd (DK)

(72) Inventor: Borrevang, Poul, 98, Taebyvej, DK-2610 Rodovre (DK)
Inventor: Petersen, Henning Borge, 93A, Kulsviervej, DK-2800 Lyngby (DK)
Inventor: Komiyama, Tetsuro, 450-31, Kuzume Washimiya-machi, Kitakatsushika-gun, Saitama-ken (JP)

(74) Representative: Brown, John David et al, FORRESTER & BOEHMERT Widenmayerstrasse 5/IV, D-8000 München 22 (DE)

## (54) Novel cephalosporin compounds, a process for the preparation thereof, pharmaceutical compositions containing the same and the use thereof as a therapeutic agent against a microorganism.

(57) The present invention relates to cephalosporins of the formula

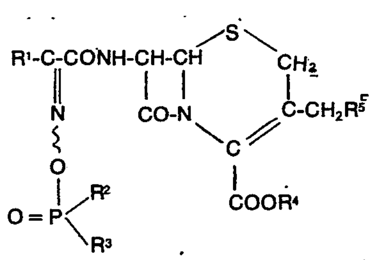

(I)

wherein $R^1$ represents an aromatic or hetero aromatic group, $R^2$ and $R^3$ represents optionally substituted alkyl, aryl, or aralkyl, a heterocyclic group, or a group of the formula $-OR^6$, wherein $R^6$ represents hydrogen, a salt forming cation, optionally substituted alkyl, aryl, or aralkyl, or a heterocyclic group, or $R^2$ and/or $R^3$ represent(s) a group of the formula $-NR^7R^8$, wherein $R^7$ and $R^8$ represents hydrogen, optionally substituted alkyl or aryl or a cycloalkyl group, or $R^7$ and $R^8$ form a heterocyclic ring, or $R^2$ and $R^3$ form a heterocyclic ring, $R^4$ represents hydrogen, a salt forming cation, or an ester forming group, and $R^5$ represents hydrogen, acyloxy, carbamoyloxy, optionally substituted pyridyl, or a group of the formula $-SR^9$, wherein $R^9$ represents alkyl or a heterocyclic group, and salts thereof. The above cephalosporins show antibiotic properties.

EP 0 018 155 A2

Title: "Novel cephalosporin compounds, a process for the preparation thereof, pharmaceutical compositions containing the same and the use thereof as a therapeutic agent against a microorganism."

THE PRESENT INVENTION relates to novel cephalosporins. The novel cephalosporins are therapeutically useful against a broad spectrum of microorganisms, especially against Proteus, Escherichia, and Klebsiella species and resistant Staphylococci species and they may be used as medicaments.

More particularly, the present invention relates to novel therapeutically useful cephalosporanic acid derivatives of the general formula

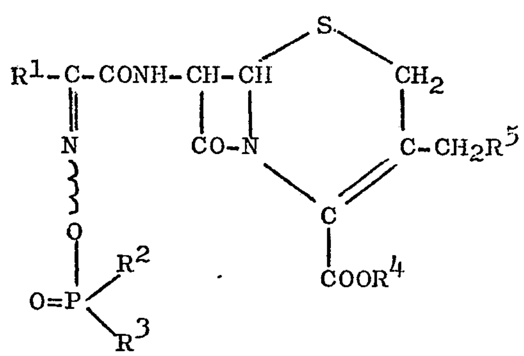

(I)

wherein $R^1$ represents an optionally substituted 5 or 6 membered aromatic or hetero aromatic group, $R^2$ and $R^3$ are the same or different and each represents lower alkyl, substituted lower alkyl, aryl, substituted aryl, aralkyl, substituted aralkyl, a 5 or 6 membered heterocyclic group containing one or two hetero atoms selected from the group consisting of nitrogen, oxygen, and sulphur, or a group of the general formula $-OR^6$, wherein $R^6$ represents hydrogen, a salt forming cation, lower alkyl, substituted

lower alkyl, aryl, substituted aryl, aralkyl, substituted aralkyl, or a 5 or 6 membered heterocyclic group containing one or two hetero atoms selected from the group consisting of nitrogen, oxygen, and sulphur, or $R^2$ and/or $R^3$ (each) represent(s) a group of the general formula $-NR^7R^8$, wherein $R^7$ and $R^8$ are the same or different and each represents hydrogen, lower alkyl, substituted lower alkyl, a 5 or 6 membered cycloalkyl group, aryl, or substituted aryl, or $R^7$ and $R^8$ together with the adjacent nitrogen atom form a 5 or 6 membered heterocyclic ring which may contain a further hetero atom selected from the group consisting of nitrogen, oxygen, and sulphur, or $R^2$ and $R^3$ together with the adjacent phosphorus atom form a 5 or 6 membered heterocyclic ring which may contain one or more further hetero atom(s) selected from the group consisting of nitrogen, oxygen, and sulphur, and which may be substituted, $R^4$ represents hydrogen, a salt forming cation, or an ester forming group, and $R^5$ represents hydrogen, acyloxy, carbamoyloxy, substituted or unsubstituted pyridyl, or a group of the general formula $-SR^9$, wherein $R^9$ represents lower alkyl or a 5 or 6 membered nitrogen containing heterocyclic group, which may contain further hetero atoms, e.g. sulphur and oxygen, and which may be substituted, whereby the wave line designates that the group

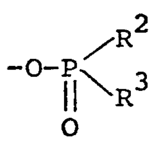

is "syn" or "anti" or mixtures thereof, and salts and hydrates thereof.

Throughout the present specification and claims, the term "lower alkyl" designates a straight or branched alkyl group, preferably containing at the most 6 carbon atoms, more preferred at the most 4 carbon atoms, preferably methyl, ethyl, propyl, and isopropyl. As examples of salt forming cations may be mentioned sodium, potassium, aluminium, calcium, or substituted ammonium, e.g. tri(lower alkyl)amino such as triethylamino, procaine, benzylamino, or dicyclohexylamino.

In case $R^2$, $R^3$, $R^6$, $R^7$, or $R^8$ is a substituted lower alkyl group, the substituent(s) may be cyano, halogen, preferably chlorine,

bromine, and fluorine, mono or di(lower alkyl)amino, preferably methylamino, ethylamino, dimethylamino, and diethylamino, lower alkoxy, preferably methoxy and ethoxy, alkoxycarbonyl, preferably methoxycarbonyl and ethoxycarbonyl, carboxy, carbamoyl, and hydroxy. Hence, examples of such substituted lower alkyl groups are 2-cyanoethyl, trifluoromethyl, 2,2,2-trifluoroethyl, 2-chloroethyl, 2,2,2-trichloroethyl, N,N-dimethylaminomethyl, 2-(methylamino)ethyl, 2-methoxyethyl, ethoxymethyl, ethoxycarbonylmethyl, carboxymethyl, carbamoylmethyl, and 2-hydroxyethyl.

It is to be understood that the statement that a group is substituted, covers groups carrying one or more substituents, preferably one or two substituents, and said substituents may be the same or different, preferably the same.

The term "aryl" designates e.g. phenyl. In case $R^2$, $R^3$, $R^6$, $R^7$, or $R^8$ is a substituted aryl group, the substituent(s) may be hydroxy, halogen, preferably chlorine, bromine, and fluorine, lower alkyl, preferably methyl and ethyl, lower alkoxy, preferably methoxy and ethoxy, mono or di(lower alkyl)amino, preferably methylamino, ethylamino, dimethylamino, and diethylamino, nitro, and cyano. Hence, examples of such substituted aryl groups are o-, m-, and p-tolyl, o-, m-, and p-hydroxyphenyl, o-, m-, and p-methoxyphenyl, 2,4-dimethoxyphenyl, 2,4-dichlorophenyl, 2,4-dibromophenyl, o-, m-, and p-bromophenyl, o-, m-, and p-fluorophenyl, p-dimethylaminophenyl, 2,4-dinitrophenyl, o-, m-, and p-cyanophenyl, 2-chloro-4-methoxyphenyl, and o-, m-, and p-nitrophenyl.

The term "aralkyl" designates an aryl(lower alkyl) group wherein the terms aryl and lower alkyl have the above meanings. As examples of aralkyl groups may be mentioned benzyl and phenethyl. In case $R^2$, $R^3$, or $R^6$ is a substituted aralkyl group, the substituent(s) may be lower alkoxy, preferably methoxy or ethoxy, halogen, preferably chlorine and bromine, nitro, di(lower alkyl)amino, preferably dimethylamino and diethylamino, and lower alkoxy, preferably methoxy and ethoxy. Hence, examples of such substituted aralkyl

groups are 4-methoxybenzyl, 4-nitrobenzyl, 4-chlorobenzyl, 4-bromobenzyl, 4-(N,N-dimethylamino)benzyl, 2-methoxybenzyl, 2-nitrobenzyl, 2,4-dinitrobenzyl, 2,4-dichlorobenzyl, 2-chloro-4-methoxybenzyl, and 3-methoxybenzyl.

As examples of a 5 or 6 membered heterocyclic group $R^2$, $R^3$, or $R^6$ containing one or two hetero atoms selected from the group consisting of nitrogen, oxygen, and sulphur may be mentioned 2-thienyl, 3-thienyl, 4-pyridyl, 2-pyridyl, 2-furyl, 3-furyl, 3-pyridazinyl, and 4-pyrimidinyl. The heterocyclic group may be substituted, and an example of a preferred substituted heterocyclic group is N-methylpiperidin-4-yl.

It is to be understood that the heterocyclic moieties stated herein may be saturated or unsaturated.

As examples of groups of the general formula $-NR^7R^8$ wherein $R^7$ and $R^8$ are the same or different groups, may be mentioned amino, N,N-dimethylamino, N,N-diethylamino, N-methyl-N-ethylamino, methylamino, isopropylamino, N,N-diisopropylamino, N-cyclohexyl-N-isopropylamino, anilino, N-methylanilino, and 2-hydroxyethylamino. As examples of groups of the general formula $-NR^7R^8$ wherein $R^7$ and $R^8$ together with the adjacent nitrogen atom form a 5 or 6 membered heterocyclic ring which may contain a further hetero atom selected from the group consisting of nitrogen, oxygen, and sulphur, may be mentioned piperidino, morpholino, 1-pyrrolyl, 1-imidazolyl, 1-pyrazolyl, *p*-isoxazin-4-yl, *o*-isoxazin-2-yl, 4-methylpiperazin-1-yl, and *p*-isothiazin-4-yl.

As examples of groups of the formula 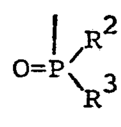 wherein $R^2$ and $R^3$ together with the adjacent phosphorus atom form a 5 or 6 membered heterocyclic ring which may contain one or more further hetero atom(s), and which may be substituted, may be mentioned 2-oxo-1,3,2-dioxaphosphorin-2-yl, 2-oxo-1,3,2-thiaoxaphosphorin-2-yl, 1,3-dimethyl-2-oxo-1,3,2-diazaphosphorin-2-yl, 2-oxo-1,3,2-dioxaphos-

phol-2-yl, 1,3-dimethyl-2-oxa-1,3,2-diazaphosphol-2-yl, 4,6-dimethyl-2-oxo-1,3,2-dioxaphosphorin-2-yl, 3-methyl-2-oxo-1,3,2-oxazophosphorin-2-yl, 1-oxophosphorinanyl, and 4-methyl-2-oxo-1,3,2-dioxaphosphol-2-yl.

As examples of groups $R^4$ may be mentioned salt forming cations such as sodium, potassium, calcium, magnesium, aluminium, or substituted ammonium, preferably tri(lower alkyl)amino, procaine, benzylamino, or dicyclohexylamino, or ester forming groups of the general formula $-\underset{\displaystyle Y}{\underset{|}{CH}}-O-CO-Z$ or $-CH(CH_3)-O-CO-O-Z$, wherein Z represents lower alkyl which may be substituted by one or more of the substituents selected from the groups consisting of lower alkoxy, lower alkylthio, and halogen(lower alkyl), and Y represents hydrogen, or Z and Y together with the adjacent group of the formula $-\underset{|}{CH}-O-CO-$ form a γ-lactone which may be substituted. As specific examples of such groups may be mentioned acetoxymethyl, tert.butylcarbonyloxymethyl, 1-(ethoxycarbonyloxy)ethyl, and o-phthalidyl. The above salt forming cations and ester forming groups are preferably pharmaceutically acceptable cations and ester groups.

As examples of acyloxy groups $R^5$ may be mentioned groups of the general formula $R^{10}COO-$ wherein $R^{10}$ represents lower alkyl, substituted lower alkyl wherein the substituent may be halogen, aryl, or aralkyl, and preferred examples of such acyloxy groups are acetoxy, benzoyloxy, phenylacetoxy, propionyloxy, and trifluoroacetoxy.

As examples of 5 or 6 membered nitrogen containing heterocyclic groups $R^9$ may be mentioned 1*H*-tetrazolyl, 2*H*-tetrazolyl, 1,2,3-triazolyl, 1,2,4-triazolyl, 1,2,3-thiadiazolyl, 1,2,4-thiadiazolyl, 1,3,4-thiadiazolyl, 1,2,5-thiadiazolyl, 1,2,3-oxadiazolyl, 1,2,4-oxadiazolyl, 1,3,4-oxadiazolyl, 1,2,5-oxadiazolyl, 2-pyridyl, 3-pyridyl, 4-pyridyl, 3-pyridazinyl, 4-pyridazinyl, 4-pyrimidinyl, and benzoethiazol-2-yl, and said heterocyclic groups may be substituted by substituents selected from the group consisting of lower alkyl, preferably methyl or ethyl, lower alkoxy, preferably methoxy, lower alkoxy(lower alkyl), preferably methoxymethyl, lower alkylthio(lower alkyl), preferably methylthiomethyl, lower alkylamino,

preferably methylamino and dimethylamino, lower alkylamino (lower alkyl), preferably methylaminomethyl, di (lower alkyl) amino (lower alkyl), preferably 2-(N,N-dimethylamino) ethyl or N,N-dimethylaminomethyl, carboxy (lower alkyl), preferably carboxymethyl and 2-carboxyethyl, carbamoyl (lower alkyl), wherein the nitrogen atom may be substituted with one or two lower alkyl group(s), preferably carbamoylmethyl, N,N-dimethylcarbamoylmethyl, and 2-(N,N-dimethylcarbamoyl) ethyl, lower alkoxycarbonyl (lower alkyl), preferably methoxycarbonylmethyl, lower alkylsulfonyl (lower alkyl), preferably methylsulfonylmethyl and 2-methylsulfonylethyl, or a group of the general formula

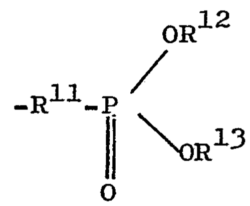

wherein $R^{11}$ represents lower alkylene, preferably methylene or ethylene, and $R^{12}$ and $R^{13}$ are the same or different and each represents hydrogen or lower alkyl, preferably methyl, ethyl, or isopropyl; hence, groups of the formula $-R^{11}-P(O)(OR^{12})(OR^{13})$ are preferably diethylphosphonylmethyl or ethylphosphonylmethyl.

As examples of preferred groups of the formula $-SR^9$ may be mentioned 1-methyltetrazol-5-ylthio, 2-methyl-1,3,4-thiadiazol-5-ylthio, methylthio, 1-[2-(N,N-dimethylamino) ethyl]tetrazol-5-ylthio, and 1-[di-ethylphosphonylmethyl] tetrazol-5-ylthio.

As examples of aromatic and hetero aromatic groups $R^1$ may be mentioned phenyl and groups containing one or two hetero atoms, which may be the same or different, selected from the group consisting of nitrogen, oxygen, and sulphur, preferably 2-thienyl, 3-thienyl, 2-furyl, 3-furyl, 4-thiazolyl, 2-pyridinyl, 3-pyridinyl, 4-pyridinyl, and 2-pyrimidinyl, and said groups may be substituted by one or two of the following substitutents: hydroxy, amino, lower alkyl, preferably methyl, lower alkylamino, preferably

methylamino, lower alkoxy, preferably methoxy, and lower alkylcarbonyloxy, preferably, acetoxy. Hence, specific examples of such substituted aromatic and hetero aromatic groups are 2-aminothiazol-4-yl, 2-amino-5-methylthiazol-4-yl, 2-methylthiazol-4-yl, 2-methyl-aminothiazol-4-yl, 2-amino-5-methoxythiazol, 5-methylthien-2-yl, 5-aminothien-2-yl, 4-hydroxyphenyl, 4-methoxyphenyl, 4-acetoxyphenyl, 4-methylaminophenyl, 2-hydroxyphenyl, 2,4-dihydroxyphenyl, 6-aminopyridin-2-yl, 6-aminopyridin-3-yl, 6-aminopyridin-4-yl, and 4-aminopyrimidin-2-yl.

A preferred subclass of compounds of the formula I is compounds wherein $R^1$ represents a group of the general formula

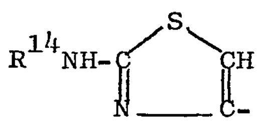

,wherein $R^{14}$ represents hydrogen or a group easily removable by hydrolysis or hydrogenolysis.

As examples of groups easily removable by hydrolysis or hydrogenolysis may be mentioned trityl, chloroacetyl, trichloroethoxycarbonyl, tert.butoxycarbonyl, benzyloxycarbonyl, trimethylsilyl, and tert.butyldimethylsilyl.

A preferred subclass of compounds of the formula I is compounds of the formula I wherein $R^1$ represents phenyl, furyl, thiazolyl, or pyrimidinyl which may be substituted by amino, $R^2$ and $R^3$ are the same or different and each represents hydroxy, phenoxy, lower alkyl, lower alkoxy which may be substituted by one, two, or three halogen atom(s), or a group of the formula $-NR^7R^8$, wherein $R^7$ and $R^8$ are the same or different and each represents hydrogen, lower alkyl, or phenyl, or wherein $R^7$ and $R^8$ together with the adjacent nitrogen atom form a 6 membered heterocyclic, saturated ring which may contain a further hetero atom, _i.e._ an oxygen atom, $R^4$ represents hydrogen or a salt forming ion, and $R^5$ represents acyloxy containing not more than 7 carbon atoms, 1-methyltetrazol-5-ylthio, or 2-methyl-1,3,4-thiadiazol-5-ylthio, and syn-isomers thereof,

and salts and hydrates thereof, preferably physiologically acceptable salts thereof.

A preferred subclass within the above subclass is compounds of the formula I wherein $R^1$ represents phenyl, furyl, thiazolyl, or pyrimidinyl which may be substituted by amino, $R^2$ and $R^3$ are the same or different and each represents hydroxy, methyl, ethyl, methoxy, ethoxy, 2,2,2-trichloroethoxy, phenoxy, N,N-diethylamino, anilino, or morpholino, $R^4$ represents hydrogen or a salt forming ion, and $R^5$ represents acyloxy containing not more than 7 carbon atoms, 1-methyltetrazol-5-ylthio, or 2-methyl-1,3,4-thiadiazol-5-ylthio, and syn-isomers thereof, and salts and hydrates thereof, preferably physiologically acceptable salts thereof.

A further preferred subclass of compounds of the formula I is compounds of the general formula I, wherein $R^1$ represents phenyl, furyl, thiazolyl, or pyrimidinyl which may be substituted by amino, $R^2$ and $R^3$ each represents hydroxy, methyl, ethyl, methoxy, ethoxy, or morpholino, $R^4$ represents hydrogen or sodium, and $R^5$ represents acetoxy, and syn-isomers thereof, and salts and hydrates thereof, preferably physiologically acceptable salts thereof.

The compounds of the formula I and salts and hydrates thereof may be prepared by the use of methods analogous to methods used for the preparation of structually similar cephalosporin derivatives. Thus, compounds of the formula I or salts or hydrates thereof can e.g., be prepared by a) reacting a compound of the general formula

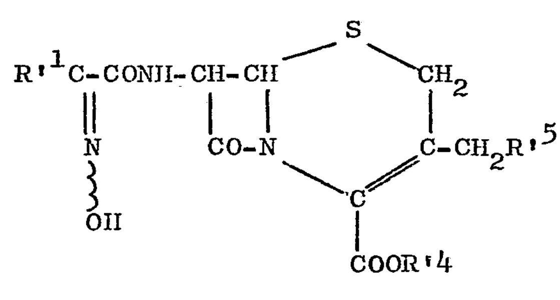

(II)

wherein $R'^1$ and $R'^5$, respectively, each has the same meaning as $R^1$ and $R^5$, respectively, or represents groups convertible thereto, and $R'^4$ represents a protecting group easily removable by hydrolysis or hydrogenolysis, with a compound of the general formula

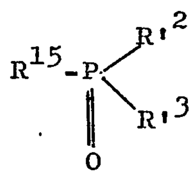

(III)

wherein $R'^2$ and $R'^3$, respectively, each has the same meaning as $R^2$ and $R^3$, respectively, or represents groups convertible thereto, and $R^{15}$ represents a leaving group commonly used as an activating group on a phosphorylating agent, or

b) reacting a compound of the general formula

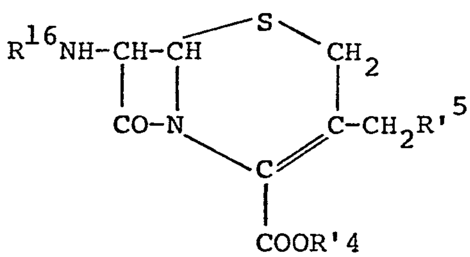

(IV)

wherein $R'^4$ and $R'^5$ each is as defined above, and $R^{16}$ represents hydrogen or a leaving group commonly connected to an amino group which is to be acylated, with a compound of the general formula

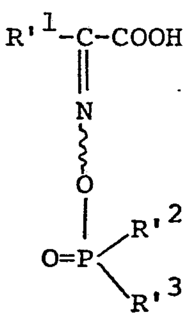

(V)

wherein $R'^1$, $R'^2$, and $R'^3$, each is as defined above, or an activated derivative thereof, and,

if necessary, subsequent to process variant a) or b) converting one or more of the groups $R'^1$, $R'^2$, $R'^3$, $R'^4$, or $R'^5$, respectively, into the corresponding groups $R^1$, $R^2$, $R^3$, $R^4$, or $R^5$, respectively, and/or, if desired, converting one or more of the groups $R^1$, $R^2$, $R^3$, $R^4$, or $R^5$, respectively, into other groups within the defini-

tions of $R^1$, $R^2$, $R^3$, $R^4$, or $R^5$, respectively, and, if desired, converting a compound of formula I into a salt or into a hydrate thereof, or, if desired, converting a salt or a hydrate of a compound of the formula I into a compound of the formula I.

The term "group convertible thereto" designates, e.g., groups wherein any hydroxy or amino group contains protecting groups, and as examples of such protecting groups may be mentioned 2,2,2-trichloroethoxycarbonyl, benzyloxycarbonyl, chloroacetyl, trityl, and a silicon or phosphorus atom carrying substituents selected from the group consisting at lower alkyl, halogen(lower alkyl), aryl, aralkyl, lower alkoxy, halogen(lower alkoxy), lower alkylthio, aralkoxy, di(lower alkyl)amino, lower alkoxyalkyl, and lower alkylenedioxy, preferably tri(lower alkyl)silyl, e.g. trimethylsilyl and tert.butyldimethylsilyl. The introduction of said groups in the above compounds and the subsequent conversion of said groups into the desired groups are carried out by methods known per se. It is essential that the protecting groups can be removed easily without any undesired influence on the parent compound and, preferably, protecting groups which can be removed by hydrolysis or hydrogenation, are used.

In case $R'^4$ represents a non pharmaceutically acceptable cation or group, it is preferably converted into a pharmaceutically acceptable cation or group by the use of methods known per se. Preferably, $R'^4$ represents an ester forming group protecting the carboxy radical which group easily can be removed after the reaction, e.g. by hydrolysis, hydrogenation, or a substitution reaction using a basic or nucleophilic reagent, and which does not interfere with the reaction. As examples of easily removable protecting groups represented by $R'^4$ may be mentioned optionally substituted benzyl, e.g. p-nitrobenzyl and p-methoxybenzyl, benzhydryl, phenacyl, e.g. p-halogenophenacyl, 2,2,2-trichloroethyl, tert.butyl, or radicals having a silicon or phosphorus atom carrying substituents selected from the group consisting of lower alkyl, halogen-(lower alkyl), aralkyl, lower alkoxy, halogen (lower alkoxy), lower alkylthio, aralkoxy, di(lower alkyl)amino, (lower alkoxy) (lower alkyl), lower alkylenedioxy, and halogen.

The reaction of a compound of the formula II with a compound of the formula III according to process variant a) is in principle a phosphorylation and, hence, it is obvious for those skilled in the art how the method is to be performed. The reaction is preferably carried out by adding a compound of the formula III which may be dissolved in an organic solvent or a mixture of organic solvents such as dichloromethane, chloroform, or tetrahydrofuran, to a compound of the formula II which may be suspended or dissolved in a solvent such as dichloromethane, chloroform, or tetrahydrofuran. The reaction may be carried out at a temperature below room temperature, preferably below 10 or 5°C, e.g. by means of an ice-bath. The reaction time depends on the reaction temperature and may be in the range between 10 minutes and 10 hours, although it may even be shorter or longer. Preferably, the reaction mixture is stirred during the reaction. The yield may be increased by the addition of acid acceptors such as pyridine, triethylamine, N,N-dimethylaniline, or epoxides, e.g. 1,2-propyleneoxide. Preferably, $R'^{4}$ represents tert.butyl, and $R^{15}$ represents halogen, preferably chlorine or bromine, p-nitrophenoxy, or imidazolyl, in the starting material.

The compounds of formula I may be recovered from the reaction mixture by means of methods known per se, e.g. by evaporation of a solvent containing said compound, by hydrolysis with an acid e.g. with trifluoroacetic acid, and by precipitation as a suitable salt or hydrate.

Compounds of the formula II can be prepared by processes known as general methods in cephalosporin chemistry or in analogy with those processes, vide, e.g., Flynn: Cephalosporins and penicillins, Acad.Press 1972, pages 76 and on and 151 and on, and M.O. Chiai et al., Chem.Pharm.

Bull., 25 (1977), page 3115 and on.

The reaction of a compound of the formula IV with a compound of

the formula V according to process variant b) is in principle an acylation and, hence, it is obvious for those skilled in the art how the method is to be performed. The reaction can be carried out in the presence of a condensing agent, e.g. a carbodiimide such as dicyclohexylcarbodiimide, optionally mixed with 1-hydroxybenzotriazole or N-hydroxysuccinimide.

A compound of the formula V may be activated by the use of methods known *per se*, e.g. by the conversion into acid halides, mixed anhydrides, and activated esters. When using acid halides, $R^{16}$ may be hydrogen, a substituted silicon or phosphorus atom, where the silicon atom may be substituted with lower alkyl, preferably trimethylsilyl, and where the phosphorus atom may be substituted with dioxoalkylene, preferably 1,3,2-dioxaphospholan-2-yl. When $R^{16}$ is hydrogen, the reaction may be performed in the presence of an acid scavenger, e.g. organic or inorganic bases or epoxides. $R'^{4}$ represents a salt forming ion or a protecting group which can be removed after the acylation reaction.

Derivatives of compounds of the formula V wherein the carboxylic group is protected, can be prepared analogously to the preparation of compounds of the formula I by means of process variant a) or by various other methods known *per se*.

It is within the ambit of those skilled in the art to judge which groups should be protected in the starting compounds when preparing compounds of the formula I or the intermediates used for the preparation thereof.

Those starting materials for which the preparation is not described herein, are either known compounds or compounds which may be prepared analogously to the preparation of known compounds or analogously to known processes.

A compound prepared by any of the above methods may be isolated and purified in a manner known *per se*, e.g. by filtration or by evaporation of the solvent and by recrystallization.

The compounds of formula I according to the present invention, and especially compounds of the formula I having syn-configuration, show interesting pharmacological properties, in particular they are effective against the following microorganisms: Staphylococcus aureus, especially resistant strains, Staphylococcus albus, Streptococcus pyogenes, Escherichia coli, Klebsiella species such as edwardsii and pneumoniae, Proteus species such as mirabilis, morganii, and vulgaris, Serratia marcescens, and Aerobacter aerogenes.

The compounds of formula I are preferably employed for therapeutic purposes in the form of amphoteric ions or non-toxic salts such as sodium, potassium, ammonium , or calcium salts. As examples of other salts that may be used, may be mentioned non-toxic, crystallising salts with organic bases such as amines, e.g. trialkylamines, procaine, and dibenzylamine.

In the treatment of bacterial infections, the antibacterial compounds of formula I can be administered topically, orally, and parenterally, in accordance with conventional procedures for antibiotic administration. They are administered in dosage units containing an effective amount of the active ingredient in combination with suitable physiologically acceptable carriers or excipients. The dosage units can be made available in the form of liquid preparations, such as solutions, suspensions, dispersions, or emulsions or in a solid form, such as powders, tablets, and capsules.

Accordingly, the invention includes pharmaceutical compositions comprising an effective amount of at least one compound of the formula I, in association with a suitable physiologically acceptable carrier or excipient. Such pharmaceutical compositions can include one or more further therapeutic ingredients in addition to a compound of the formula I. Herein, the term "effective amount" in relation to the compounds of the formula I designates an amount which is sufficient to destroy or inhibit the growth of susceptible microorganisms when administered in the usual manner, in other words an amount which is sufficient to control the

growth of bacteria. The magnitude of an effective amount can easily be determined by those skilled in the art through standard procedures for determining the relative activity of antibacterial agents when utilized against susceptible organisms via the various available routes of administration. A preferred formulation for parenteral use may contain 250 - 1000 mg of a compound of the formula I, preferably a soluble salt, per unit dosage which may be administered 1 - 4 times per day, depending on the diagnosis of the patient. A preferred tablet formulation for oral administration may contain 250 - 1000 mg of a compound of the formula I per unit dosage which may be administered 1 - 4 times per day.

Suitable carriers and excipients may be any convenient physiologically acceptable ingredient which can serve to facilitate administration of the compound of the formula I. Carriers may provide some ancillary function such as that of a diluent, flavour-masking agent, binding agent, sustained release agent, or stabilizer. As examples of carriers may be mentioned water, which may contain gelatin, acacia, alginate, dextran, polyvinylpyrrolidone, sodium carboxymethyl cellulose, aqueous ethanol, syrup, isotonic saline, isotonic glucose, starch, lactose, or any other material commonly used in the pharmaceutical and veterinary industry.

In connection with the nomenclature used concerning the phosphorus moiety of the compounds stated herein reference is *inter alia* made to Houben-Weyl XII/1, 1 - 14.

The process for the preparation of the above-mentioned novel compounds is illustrated by the following examples which, however, are not to be construed as limiting. The examples especially illustrate the preferred embodiments of the process. However, the yields obtained can in some cases be increased substantially.

Example I.

a) tert.Butyl 7β-(γ-bromoacetylacetamido)cephalosporanate.

To a solution of diketene (2.4 g) in methylene chloride (10 ml), a solution of bromine (4.1 g) in methylene chloride (10 ml) was added dropwise at a temperature below -20°C. The acid bromide obtained in methylene chloride solution was added dropwise to a solution of tert. butyl 7β-aminocephalosporanate (9.6 g) and triethylamine (4.0 ml) in methylene chloride at a temperature below -20°C with stirring. After heating to room temperature during 1 hour, the reaction mixture was washed with water, with 1N hydrochloric acid, with an aqueous solution of sodium bicarbonate, and with brine, and was thereafter dried over sodium sulphate. By removal of the solvent 13.5 g of tert. butyl 7β-(γ-bromoacetylacetamido)cephalosporanate was obtained (yield: 94%).

IR ν (max., KBr) $cm^{-1}$: 1780, 1740, 1730, 1680, and 1660 (C=O). NMR (in $CDCl_3$), δ : 1.56 (9H, s, $-C(CH_3)_3$), 2.08 (3H, s, $-COCH_3$), 3.32 (1H, d, J = 20 Hz, $C_2$-H), 3.60 (1H, d, J = 20 Hz, $C_2$-H), 3.72 (2H, s, $-COCH_2CO-$), 4.04 (2H, s, $BrCH_2CO-$), 4.78 (1H, d, J = 12 Hz, $C_3-CH_2-$), 4.96 (1H, d, $C_6$-H), 5.12 (1H, d, J = 12 Hz, $C_3-CH_2-$), 5.80 (1H, dd, $C_7$-H), and 7.60 (1H, d, -NH).

b) tert.Butyl 7β-(2-bromoacetyl-2-hydroxyiminoacetamido)cephalosporanate.

To a solution of tert.butyl 7β-(γ-bromoacetylacetamido) cephalosporanate (13.5 g) in glacial acetic acid (130 ml), sodium nitrite (1.8g) in water (35 ml) was added dropwise at a temperature below

$7^{o}C$ with stirring. After stirring for 2 hours at a temperature below $7^{o}C$ water (200 ml) was added and the mixture was extracted with methylene chloride (200 ml). The organic layer was washed with water (200 ml x 3) and dried over sodium sulphate. The solvent was evaporated, whereby 13.3 g of tert.butyl 7β-(2-bromoacetyl-2-hydroxyiminoacetamido)cephalosporanate was obtained (yield: 93%).

IR ν (max., KBr) $cm^{-1}$: 1780, 1740, 1725, and 1710 (C=O).
NMR (in $CDCl_3$), δ : 1.55 (9H, s, $-C(CH_3)_3$), 2.05 (3H, s, $-COCH_3$), 3.36 (1H, d, J = 16 Hz, $C_2$-H), 3.60 (1H, d, J = 16 Hz, $C_2$-H), 4.50 (2H, s, $BrCH_2CO-$), 4.69 (1H, d, J = 12 Hz, $C_3-CH_2-$), 5.00 (1H, d, J = 6 Hz, $C_6$-H), 5.08 (1H, d, J = 12 Hz, $C_3-CH_2-$), and 5.80 (1H, dd, J = 6 Hz, J = 10 Hz, $C_7$-H).

c) tert.Butyl 7β-[2-(2-aminothiazol-4-yl)-2-hydroxyiminoacetamido]cephalosporanate (syn-form).

A mixture of tert.butyl 7β-(2-bromoacetyl-2-hydroxyiminoacetamido)cephalosporanate (10.1 g) and thiourea (1.3 g) in ethanol (100 ml) was heated at $50^{o}C$ for 20 minutes with stirring. The ethanol was evaporated, whereby a crude product was obtained, which was extracted with ethyl acetate and washed with water. The pH value of the water layer was adjusted to 6.0 with an aqueous sodium bicarbonate solution and extracted with ethyl acetate. The combined ethyl acetate layers were washed with brine and dried over sodium sulphate. The solvent was evaporated, whereby a crude product was obtained, which was crystallized from acetone to yield 5.4 g of white crystals (yield: 56%). The compound is syn-form.

IR ν (max., KBr) $cm^{-1}$: 1780, 1740, 1735, and 1680 (C=O).
NMR (in DMSO-$d_6$), δ : 1.45 (9H, s, $-C(CH_3)_3$), 2.00 (3H, s, $-COCH_3$), 3.31 (1H, d, J = 16 Hz, $C_2$-H), 3.55 (1H, d, J = 16 Hz, $C_2$-H), 4.50 (1H, d, J = 14 Hz, $C_3-CH_2-$), 4.82 (1H, d, J = 14 Hz,

$C_3$-$CH_2$-), 5.20 (1H, d, J = 4 Hz, $C_6$-H), 5.68 (1H, dd, J = 4 Hz, J = 8 Hz, $C_7$-H), 6.50 (1H, s, thiazole-5-H), 6.96 (2H, broad s, $H_2N$-), and 9.22 (1H, d, J = 8 Hz, -NH).

d) tert.Butyl 7β-[2-(2-aminothiazol-4-yl)-2-diethylphosphoryloxyiminoacetamido]cephalosporanate.

tert.Butyl 7β-[2-(2-aminothiazol-4-yl)-2-hydroxyiminoacetamido]-cephalosporanate (300 mg) and triethylamine (120 mg) were dissolved in methylene chloride (10 ml) and the mixture was stirred in an ice-water bath. Diethyl phosphorochloridate (210 mg) in methylene chloride (2 ml) was added dropwise at a temperature below 10°C. Stirring and cooling were maintained for 30 minutes. The reaction mixture was washed with water, with 0.5N hydrochloric acid, with water, and with brine, dried over sodium sulphate, and evaporated to dryness <u>in vacuo</u>. The residue was triturated with diethyl ether to yield 250 mg of tert.butyl 7β-[2-(2-aminothiazol-4-yl)-2-diethylphosphoryloxyiminoacetamido]cephalosporanate (yield: 65%).

IR ν (max., KBr) $cm^{-1}$: 1785, 1740, 1730, and 1690 (C=O).
NMR (in $CDCl_3$), δ : 1.20 - 1.60 (6H, m, -P(O)($OCH_2C\underline{H}_3$)$_2$, 1.60 (9H, s, -C($CH_3$)$_3$), 2.08 (3H, s, -$COCH_3$), 3.50 (2H, dd, $C_2$-H), 4.00 - 4.40 (4H, m, -P(O)($OC\underline{H}_2CH_3$)$_2$), 4.92 (2H, dd, $C_3$-$CH_2$-), 5.12 (1H, d, $C_6$-H), 6.00 (1H, dd, $C_7$-H), 6.85 (1H, s, thiazole-5-H), 7.68 (2H, broad s, $NH_2$), and 9.30 (1H, broad s, NH).

e) 7β-[2-(2-Aminothiazol-4-yl)-2-diethylphosphoryloxyiminoacetamido]cephalosporanic acid.

tert.Butyl 7β-[2-(2-aminothiazol-4-yl)-2-diethylphosphoryloxyiminoacetamido]cephalosporanate (250 mg) was dissolved in 5 ml of trifluoroacetic acid and the mixture was stirred in an ice-water bath under a nitrogen atmosphere for 2 hours. After evaporation of the trifluoroacetic acid, the residual oil was dissolved in ethyl

acetate, washed with water, and dried over sodium sulphate. The solvent was evaporated, whereby an oil was obtained, which was triturated with diethyl ether to yield 128 mg of 7β-[2-(2-aminothiazol-4-yl)-2-diethylphosphoryloxyiminoacetamido]cephalosporanic acid as a white powder (yield: 56%).

IR ν (max., KBr) $cm^{-1}$: 1785, 1730, and 1680 (C=O).
NMR (in acetone-$d_6$), δ : 1.20 - 1.60 (6H, m, $-P(O)(OCH_2C\underline{H}_3)_2$), 2.05 (3H, s, $COCH_3$), 3.72 (2H, dd, $C_2$-H), 4.20 - 4.50 (4H, m, $-P(O)(OC\underline{H}_2CH_3)_2$), 5.08 (2H, dd, $C_3$-$CH_2$), 5.38 (1H, d, $C_6$-H), 6.10 (1H, dd, $C_7$-H), 7.32 (1H, s, thiazole-5-H), 7.72 (3H, broad, $NH_2$ + COOH), and 9.28 (1H, d, NH).

Example 2.

a) tert.Butyl 7β-[2-(2-aminothiazol-4-yl)-2-dimethylphosphoryloxyiminoacetamido]cephalosporanate.

The compound was prepared analogously to the process described in Example 1d and was obtained as a powder (yield: 94%).

IR ν (max., KBr) $cm^{-1}$: 1785, 1740, 1730, and 1680 (C=O).
NMR (in acetone-$d_6$), δ : 1.50 (9H, s, $-C(CH_3)_3$), 3.40 - 4.00 (8H, m, $-P(O)(OCH_3)_2$ + $C_2$-H), 7.16 (1H, s, thiazole-5-H), and 9.20 (1H, d, NH).

b) 7β-[2-(2-Aminothiazol-4-yl)-2-dimethylphosphoryloxyiminoacetamido]cephalosporanic acid.

The compound was prepared analogously to the process described in Example 1e and was obtained as a powder (yield: quantitatively).

IR ν (max., KBr) $cm^{-1}$: 1780, 1730, and 1680 (C=O).
NMR (in DMSO-$d_6$), δ : 2.00 (3H, s, $-COCH_3$), 3.40 - 3.80 (8H, m, $-P(O)(OCH_3)_2$ + $C_2$-H), 4.76 (2H, dd, $C_3$-$CH_2$), 5.08 (1H, d, $C_6$-H), 5.70 (1H, m, $C_7$-H), 6.90 (1H, s, thiazole-5-H), and 9.70 (1H, broad, NH).

Example 3.

a) tert.Butyl 7β-[2-(2-aminothiazol-4-yl)-2-bis(2,2,2-trichloroethyl) phosphoryloxyiminoacetamido]cephalosporanate.

The compound was prepared analogously to the process described in Example 1d and was obtained as a powder (yield: 77%).

IR ν (max., KBr) $cm^{-1}$: 1780, 1760, 1720 (C=O), 1240 (P=O), 1150 (C-O-(P)), and 1030 (P-O-(C)).
NMR (in $CDCl_3$), δ : 1.50 (9H, s, $C(CH_3)_3$), 2.08 (3H, s, $-COCH_3$), 3.44 (2H, dd, $C_2$-H), 4.60 - 5.20 (7H, m, $C_3-CH_2$, $-P(O)-OC\underline{H}_2CCl_3$ x 2, $C_6$-H), 5.70 (1H, m, $C_7$-H), and 7.24 (1H, s, thiazole-5-H, overlapped with $CHCl_3$).

b) 7β-[2-(2-Aminothiazol-4-yl)-2-bis(2,2,2-trichloroethyl)phosphoryloxyiminoacetamido]cephalosporanic acid.

The compound was prepared analogously to the process described in Example 1e and was obtained as a powder (yield: 39%).

IR ν (max., KBr) $cm^{-1}$: 1780, 1730, and 1690 (C=O).
NMR (in DMSO-$d_6$), δ : 2.00 (3H, s, $COCH_3$), 3.50 (2H, dd, $C_2$-H), 4.50 (4H, d, $CCl_3C\underline{H}_2$-), 4.70 - 5.30 (3H, m, $C_3-CH_2$, $C_6$-H), 5.80 (1H, m, $C_7$-H), 7.10 (1H, s, thiazole-5-H), and 10.00 (1H, d, NH).

Example 4.

a) tert.Butyl 7β-[2-(2-aminothiazol-4-yl)-2-dimorpholinophosphoryloxyiminoacetamido]cephalosporanate.

The compound was prepared analogously to the process described in Example 1d and was obtained as a powder (yield: 75%).

IR $\nu$ (max., KBr) $cm^{-1}$: 1775, 1720, and 1680 (C=O).
NMR (in DMSO-$d_6$) $\delta$ : 1.50 (9H, s, $C(CH_3)_3$), 2.00 (3H, s, $COCH_3$), 3.10 and 3.60 (16H, m, morpholino x 2), 4.90 (2H, dd, $C_3$-$CH_2$), 5.02 (1H, d, $C_6$-H), 5.84 (1H, dd, $C_7$-H), 6.80 (1H, s, thiazole-5-H), and 9.00 (1H, d, NH).

b) 7β-[2-(2-Aminothiazol-4-yl)-2-dimorpholinophosphoryloxyiminoacetamido]cephalosporanic acid.

The compound was prepared analogously to the process described in Example 1e and was obtained as a brownish powder (yield: 92%).

IR $\nu$ (max., KBr) $cm^{-1}$: 1775, 1720, and 1660 (C=O).
NMR (in DMSO-$d_6$) $\delta$ : 2.00 (3H, s, $COCH_3$), 3.05 and 3.50 (16H, m, morpholino x 2), 4.84 (2H, dd, $C_3$-$CH_2$), 5.15 (1H, d, $C_6$-H), 5.80 (1H, d, $C_7$-H), 7.15 (1H, s, thiazole-5-H), and 9.80 (1H, d, NH).

Example 5.

a) tert.Butyl 7β-[2-(2-aminothiazol-4-yl)-2-dianilinophosphoryloxyiminoacetamido]cephalosporanate.

The compound was prepared analogously to the process described in Example 1d and was obtained as a powder (yield: quantitatively).

IR $\nu$ (max., KBr) $cm^{-1}$: 1765, 1720, and 1680 (C=O).
NMR (in $CDCl_3$) $\delta$ : 1.52 (9H, s, $-C(CH_3)_3$), 2.04 (3H, s, $COCH_3$), 3.50 (2H, broad, $C_2$-H), 5.00 (2H, dd, $C_3$-$CH_2$), 5.12 (1H, d, $C_6$-H), 5.88 (1H, broad, $C_7$-H), and 6.60 - 7.20 (11H, m, phenyl x 2 + thiazole-5-H).

b) 7β-[2-(2-Aminothiazol-4-yl)-2-dianilinophosphoryloxyiminoacetamido]cephalosporanic acid.

The compound was prepared analogously to the process described in Example 1e and was obtained as a powder (yield: 81%).

IR $\nu$ (max., KBr) $cm^{-1}$: 1780, 1720, and 1660 (C=O).
NMR (in DMSO-$d_6$) $\delta$ : 1.04 (3H, s, $COCH_3$), 3.44 (2H, d, $C_2$-H), 4.84 (2H, dd, $C_3$-$CH_2$), 5.10 (1H, d, $C_6$-H), 5.70 (1H, broad, $C_7$-H), 7.00 - 7.24 (11H, m, phenyl x 2 + thiazole-5-H), and 9.80 (1H, d, $C_7$-NH).

Example 6.

a) tert.Butyl 7β-[2-(2-aminothiazol-4-yl)-2-diphenylphosphoryloxyiminoacetamido]cephalosporanate.

The compound was prepared analogously to the process described in Example 1d and was obtained as a powder (yield: 91%).

IR $\nu$ (max., KBr) $cm^{-1}$: 1785, 1740, 1720, and 1690 (C=O).
NMR (in $CDCl_3$) $\delta$ : 1.50 (9H, s, $-C(CH_3)_3$), 2.00 (3H, s, $-COCH_3$), 3.30 (2H, d, J = 20 Hz, $C_2$-H), 4.86 (2H, dd, $C_3$-$CH_2$), 4.96 (1H, d, $C_6$-H), 5.70 (1H, dd, $C_7$-H), 6.84 (1H, s, thiazole-5-H), and 7.10 - 7.20 (10H, m, phenyl x 2).

b) 7β-[2-(2-Aminothiazol-4-yl)-2-diphenylphosphoryloxyiminoacetamido]cephalosporanic acid.

The compound was prepared analogously to the process described in Example 1e and was obtained as a powder(yield: quantitatively).

IR $\nu$ (max., KBr) $cm^{-1}$: 1780, 1720, and 1680 (C=O).
NMR (in DMSO-$d_6$) $\delta$ : 2.00 (3H, s, $COCH_3$), 3.50 (2H, dd, J = 20 Hz, $C_2$-H), 4.94 (2H, dd, $C_3$-$CH_2$), 5.16 (1H, d, J = 4 Hz, $C_6$-H), 5.80 (1H, dd, J = 4 Hz, J = 8 Hz, $C_7$-H), 7.08 (1H, s, thiazole-5-H), and 7.20 - 7.40 (10H, m, phenyl x 2).

Example 7.

a) tert.Butyl 7β-[2-(2-aminothiazol-4-yl)-2-methylmethylphosphonyloxyiminoacetamido]cephalosporanate.

The compound was prepared analogously to the process described in Example 1d and was obtained as a pale brownish powder (yield: 90%).

b) 7β-[2-(2-Aminothiazol-4-yl)-2-methylmethylphosphonyloxyiminoacetamido]cephalosporanic acid.

The above prepared ester was deesterified as described in Example 1e and the acid was obtained as a pale brownish powder (yield: 69%).

IR ν (max., KBr)$cm^{-1}$: 1780 and 1720 (C=O).

NMR (in DMSO-$d_6$) δ : 1.50 - 1.80 (3H, m, P-$CH_3$), 2.00 (3H, s, $COCH_3$), 3.40 - 3.80 (5H, m and d, $C_2$-H and $POCH_3$), 4.80 (2H, dd, $C_3$-$CH_2$-), 5.12 (1H, d, $C_6$-H), 5.74 (1H, dd, $C_7$-H), 6.96 (1H, s, thiazole-5-H), and 9.76 (1H, d, NH).

Example 8.

a) tert.Butyl 7β-[2-(2-aminothiazol-4-yl)-2-methylphosphoryloxyiminoacetamido]cephalosporanate.

The compound was prepared analogously to the process described in Example 1d using methyl(triethylsilyl)phosphoryl chloride as the phosphorylating agent. The product was obtained as a light brown powder (yield: 26%).

IR ν (max., KBr) $cm^{-1}$: 1770, 1720, and 1670 (C=O).

NMR (in $CDCl_3$ + $CD_3OD$) δ : 1.50 (9H, s,$C(CH_3)_3$), 2.08 (3H, s, $OCOCH_3$), 3.30 - 3.70 (5H, m, $POCH_3$ and $C_2$-H), 4.68 - 5.20 (3H, m, $C_3$-$CH_2$- and $C_7$-H), and 6.80 (1H, s, thiazole-5-H).

b) 7β-[2-(2-Aminothiazol-4-yl)-2-methylphosphoryloxyiminoacetamido]-cephalosporanic acid.

The compound was prepared analogously to the process described in Example 1e and the acid was obtained after preparative TLC (thin layer chromatography) as a powder (yield: 20%).

IR ν (max., KBr) $cm^{-1}$: 1775, 1730, and 1680 (C=O).
MIC value against P. vulgaris ATCC 6897: 0.2 μg/ml.

Example 9.

a) tert.Butyl 7β-[2-(2-aminothiazol-4-yl)-2-methylethylphosphonyloxyiminoacetamido]cephalosporanate.

The compound was prepared analogously to the process described in Example 1d and was obtained as a light yellow powder (yield: quantitative).

NMR (in $CDCl_3$) δ : 1.58 (9H, s, $C(CH_3)_3$), 1.20 (3H, m, $PCH_2C\underline{H}_3$), 2.00 (3H, s, $OCOCH_3$), 3.50 (2H, dd, $C_2$-H), 1.95 (2H, m, $PC\underline{H}_2CH_3$), 3.80 (3H, d, $POCH_3$), 4.95 (2H, dd, $C_3$-$CH_2$), 5.20 (1H, d, $C_6$-H), 6.00 (1H, dd, $C_7$-H), 6.90 (1H, s, thiazole-5-H), 7.80 (2H, broad s, $NH_2$), and 9.70 (1H, broad d, NH).

b) 7β-[2-(2-Aminothiazol-4-yl)-2-methylethylphosphonyloxyiminoacetamido]cephalosporanic acid.

The compound was prepared analogously to the process described in Example 1e and was obtained as a powder (yield: 85%).

IR ν (max., KBr) $cm^{-1}$: 1780, 1720, 1680, and 1650 (C=O).
NMR (in DMSO-$d_6$) δ : 1.00 and 1.20 (3H, dt, $PCH_2C\underline{H}_3$), 2.00 (3H, s, $OCOCH_3$), 1.95 (2H, m, $PC\underline{H}_2CH_3$), 3.60 (2H, dd, $C_2$-H), 3.64 (3H, d, $POCH_3$), 4.80 (2H, dd, $C_3$-$CH_2$), 5.20 (1H, d, $C_6$-H), 5.83 (1H, dd, $C_7$-H), 7.00 (1H, s, thiazole-5-H), 7.50 (2H, broad s, $NH_2$), and 9.95 (1H, d, NH).

Example 10.

a) tert.Butyl 7β-[2-(2-aminothiazol-4-yl)-2-phenylethylphosphonyloxyiminoacetamido]cephalosporanate.

The compound was prepared analogously to the process described in Example 1d and was obtained as a light yellow powder (yield: quantitative).

NMR (in $CDCl_3$) δ : 1.10 and 1.24 (3H, dt, $PCH_2C\underline{H}_3$), 1.54 (9H, s, $C(CH_3)_3$), 2.06 (3H, s, $OCOCH_3$), 3.4 (2H, dd, $C_2$-H), 4.65 - 5.30 (3H, m, $C_3$-$CH_2$ and $C_6$-H), 5.90 (1H, m, $C_7$-H), and 6.80 - 7.50 (6H, m, aromatic protons).

b) 7β-[2-(2-Aminothiazol-4-yl)-2-phenylethylphosphonyloxyiminoacetamido]cephalosporanic acid.

The compound was prepared analogously to the process described in Example 1e and was obtained as a light coloured powder (yield: 90%).

IR ν (max., KBr) $cm^{-1}$: 1770, 1720, and 1660 (C=O).
NMR (in DMSO-$d_6$) δ : 1.04 and 1.26 (3H, dt, $PCH_2C\underline{H}_3$), 2.02 (3H, s, $OCOCH_3$), 3.52 (2H, dd, $C_2$-H), 4.85 (2H, dd, $C_3$-$CH_2$), 5.16 (1H, d, $C_6$-H), and 7.00 - 7.48 (6H, m, aromatic protons).

Example 11.

a) tert.Butyl 7β-[2-(2-aminothiazol-4-yl)-2-(N,N-diethylamino)methylphosphonyloxyiminoacetamido]cephalosporanate.

The compound was prepared analogously to the process described in Example 1d and was obtained as a yellow powder (yield: 98.2%).

NMR (in $CDCl_3$) δ : 0.9 - 1.3 (6H, m, $N(CH_2C\underline{H}_3)_2$), 1.58 (9H, s, $C(CH_3)_3$), 1.63 (3H, d, $PCH_3$), 2.10 (3H, s, $OCOCH_3$), 2.80 - 3.35 (4H, m, $N(C\underline{H}_2CH_3)_2$), 3.52 (2H, dd, $C_2$-H), 5.0 (2H, dd, $C_3$-$CH_2$), 5.16 (1H, d, $C_6$-H), 6.0 (1H, dd, $C_7$-H), and 6.85 (1H, s, thiazole-5-H).

b) 7β-[2-(2-Aminothiazol-4-yl)-2-(N,N-diethylamino)methylphosphonyloxyiminoacetamido]cephalosporanic acid.

The compound was prepared analogously to the process described in Example 1c and was obtained as a light tan powder (yield: quantitative).

IR ν (max., KBr) $cm^{-1}$: 1775 (β-lactam C=O).
NMR (in $DMSO-d_6$) δ : 1.20 (6H, t, $N(CH_2CH_3)_2$), 1.50 (3H, d, $PCH_3$), 2.07 (3H, s, $OCOCH_3$), 2.8 - 3.3 (4H, m, $PN(CH_2CH_3)_2$), 3.60 (2H, dd, $C_2$-H), 4.92 (2H, dd, $C_3$-$CH_2$), 5.26 (1H, d, $C_6$-H), 5.90 (1H, dd, $C_7$-H), and 7.10 (1H, s, thiazole-5-H).

Example 12.

tert.Butyl 7β-[2-(2-aminothiazol-4-yl)-2-ethylmethylphosphonyloxyiminoacetamido]cephalosporanate.

The compound was prepared analogously to the process described in Example 1d and was obtained as a light brown powder (yield: 83%).

NMR (in $CDCl_3$) δ : 1.10 - 1.70 (6H, m, 2 x $CH_3$), 1.53 (9H, s, $C(CH_3)_3$), 2.04 (3H, s, $OCOCH_3$), 3.50 (2H, dd, $C_2$-H), 4.0 - 4.10 (2H, m, $POCH_2$), 4.93 (2H, dd, $C_3$-$CH_2$), 5.16 (1H, d, $C_6$-H), 6.04 (1H, dd, $C_7$-H), and 6.50 (1H, s, thiazole-5-H).

b) 7β-[2-(2-Aminothiazol-4-yl)-2-ethylmethylphosphonyloxyiminoacetamido]cephalosporanic acid.

The compound was prepared analogously to the process described in Example 1e and was obtained as a yellow powder (yield: 72%).

IR ν (max., KBr) $cm^{-1}$: 1780 and 1680 (C=O).
NMR (in $DMSO-d_6$) δ : 1.22 (3H, t, $CH_2CH_3$), 1.56 (3H, d, $PCH_3$), 2.00 (3H, s, $OCOCH_3$), 3.52 (2H, dd, $C_2$-H), 3.90 - 4.20 (2H, m, $POCH_2CH_3$), 4.82 (2H, dd, $C_3$-$CH_2$), 5.13 (1H, d, $C_6$-H), 5.79 (1H, dd, $C_7$-H), 7.00 (1H, s, thiazole-5-H), and 9.80 - 10.00 (1H, broad, NH).

Example 13

a) tert.Butyl 7β-[2-(fur-2-yl)-2-hydroxyiminoacetamido]cephalosporanate.

Into a solution of dichloroacetyl chloride (380 mg) in tetrahydrofuran (2 ml), 2-(fur-2-yl)-2-hydroxyiminoacetic acid (155 mg) was added in portions, and the mixture was stirred at room temperature for 2 hours. After removal of the tetrahydrofuran by evaporation, the residual oil was washed well with petroleum ether to yield 100 mg of a gum, which was suspended in methylene chloride (5 ml). Phosphorus pentachloride (78 mg) was added to the mixture and the mixture was stirred for 30 minutes. The solvent was removed by evaporation to yield the acid chloride as an oil. The oil was dissolved in 2 ml of methylene chloride.

To a solution of tert.butyl 7β-aminocephalosporanate (125 mg) and triethylamine (38 mg) in methylene chloride (4 ml), the previously prepared acid chloride in methylene chloride was added dropwise and the mixture was stirred for 40 minutes in an ice bath and stirred at room temperature for 40 minutes. After removal of the solvent by evaporation, the residual oil was extracted with ethyl acetate, washed with water, with 1N hydrochloric acid, with an aqueous solution of sodium bicarbonate, and with water, dried over sodium sulphate, and evaporated to give 90 mg of a crude product. Preparative thin layer chromatography (TLC) (silica gel, toluene/ethyl acetate = 1/1) yielded 33 mg of tert.butyl 7β-[2-(fur-2-yl)-2-hydroxyiminoacetamido]cephalosporanate.

NMR (in $CDCl_3$) δ : 1.52 (9H, s, $-C(CH_3)_3$), 2.04 (3H, s, $-COCH_3$), 3.24 (1H, d, J = 18 Hz, $C_2$-H), 3.52 (1H, d, J = 18 Hz, $C_2$-H), 4.65 (1H, d, J = 12 Hz, $C_3-CH_2-$), 4.92 (1H, d, $J_{6-7}$ = 4 Hz, $C_6$-H), 4.96 (1H, d, J = 12 Hz, $C_3-CH_2-$), 5.84 (1H, dd, $J_{6-7}$ = 4 Hz, $J_{7-NH}$ = 8 Hz, $C_7$-H), 6.30, 6.70, 7.30 (3H, m, aromatic protons), and 7.76 (1H, d, $C_{7-NH}$ = 8 Hz, NH).

b) tert.Butyl 7β-[2-(fur-2-yl)-2-diethylphosphoryloxyiminoacetamido]cephalosporanate.

To a solution of the oxime prepared according to subsection a) (28 mg) and DABCO® (1,4-diazabicyclo[2,2,2]octane) (8 mg) in tetrahydrofuran (5 ml), diethyl phosphorochloridate (21 mg) was added and the mixture was

stirred at room temperature for 3.5 hours. After removal of the tetrahydrofuran by evaporation, the residual oil was extracted with ethyl acetate, washed with water, with 1N hydrochloric acid, and with brine, dried over sodium sulphate, and evaporated to yield a crude product (43 mg). Preparative TLC (silica gel, benzene/ethyl acetate = 1/1) yielded 18 mg of the pure product (yield: 46%).

IR ν (max., KBr) $cm^{-1}$: 1780, 1740, 1730, 1690 (C=O), 1260 (P=O), and 1030 (P-O-C).
NMR (in $CDCl_3$) δ : 1.36 (6H, m, $-P(O)(OCH_2C\underline{H}_3)_2$), 1.54 (9H, s, $-C(CH_3)_3$), 3.34 (1H, d, J = 20 Hz, $C_2$-H), 3.59 (1H, d, J = 20 Hz, $C_2$-H), 4.00 - 4.40 (4H, m, $-P(O)(OC\underline{H}_2CH_3)_2$), 4.79 (1H, d, J = 14 Hz, $C_3-CH_2-$), 5.05 (1H, d, $J_{6-7}$ = 4 Hz, $C_6$-H), 5.08 (1H, d, J = 14 Hz, $C_3-CH_2-$), 5.86 (1H, dd, $J_{6-7}$ = 4 Hz, $J_{7-NH}$ = 8 Hz, $C_7$-H), 6.50, 6.88, 7.60 (3H, m, aromatic protons), and 7.68 (1H, d, $J_{7-NH}$ = 8 Hz, NH).

c) 7β-[2-(Fur-2-yl)-2-diethylphosphoryloxyiminoacetamido]cephalosporanic acid.

The tert.butyl ester prepared according to subsection b) (65 mg) was hydrolysed with trifluoroacetic acid (1.0 ml) in an ice bath for 1 hour under nitrogen atmosphere to yield 39 mg of the free acid (yield: 65%).

IR ν (max., KBr) $cm^{-1}$: 1780, 1730, 1690 (C=O), 1230 (P=O), and 1030 (P-O-C).
NMR (in $CDCl_3$) δ : 1.20 - 1.40 (6H, m, $-P(O)(OCH_2C\underline{H}_3)_2$), 2.04 (3H, s, $COCH_3$), 3.28 (1H, d, J = 18 Hz, $C_2$-H), 3.60 (1H, d, J = 18 Hz, $C_2$-H), 4.00 - 4.30 (4H, m, $-P(O)(OC\underline{H}_2CH_3)_2$), 4.84 (1H, d, J = 12 Hz, $C_3-CH_2-$), 5.04 (1H, d, $J_{6-7}$ = 4 Hz, $C_6$-H), 5.08 (1H, d, J = 12 Hz, $C_3-CH_2-$), 5.80 (1H, dd, $J_{6-7}$ = 4 Hz, $J_{7-NH}$ = 8 Hz, $C_7$-H), 6.50, 6.90, 7.56 (3H, m, aromatic protons), and 8.40 (1H, d, $J_{7-NH}$ = 8 Hz, NH).

Example 14.

a) 2-(Dichloroacetoxyimino)phenylacetic acid.

To a solution of dichloroacetyl chloride (450 mg) in methylene chloride (5 ml), 2-hydroxyiminophenylacetic acid (140 mg) was added in portions at a temperature below 5°C, and the mixture was stirred at room temperature for 20 minutes. The crystallized product was filtered off and washed with petroleum ether to yield 175 mg of the product as needles (yield: 63.4%) with a melting point of 106 - 107°C (decomposition).

IR ν (max., KBr) $cm^{-1}$: 1760 (C=O).

b) tert.Butyl 7β-(2-phenyl-2-hydroxyiminoacetamido)cephalosporanate.

To a suspension of the acid prepared according to subsection a) (525 mg) in methylene chloride phosphorus pentachloride (400 mg) was added in portions at a temperature below 5°C, and the mixture was stirred for 40 minutes. After removal of the solvent in vacuo, the residual oil was dissolved in benzene. The benzene was evaporated off to yield the acid chloride as an oil.

To a solution of tert.butyl 7β-aminocephalosporanate (625 mg) and triethylamine (200 mg) in methylene chloride (15 ml), the previously prepared acid chloride in methylene chloride (15 ml) was added at a temperature below 5°C and the mixture was stirred for 30 minutes. The methylene chloride was evaporated off, and the residue was extracted with ethyl acetate, washed with water and with brine and dried over sodium sulphate. The ethyl acetate was evaporated off to yield 1040 mg of a crude product. Purification by preparative TLC (silica gel, chloroform/methanol = 10/1) yielded 320 mg of the pure product (yield: 35.4%).

IR ν (max., $CHCl_3$) $cm^{-1}$: 1690, 1730, and 1790 (C=O).

NMR (in $CDCl_3$) $\delta$ : 1.51 (9H, s, $-C(CH_3)_3$), 2.06 (3H, s, $COCH_3$), 3.25 (1H, d, J = 18 Hz, $C_2$-H), 3.55 (1H, d, J = 18 Hz, $C_2$-H), 4.75 (1H, d, J = 14 Hz, $C_3-CH_2-$), 5.00 (1H, d, $J_{6-7}$ = 4 Hz, $C_6$-H), 5.05 (1H, d, J = 14 Hz, $C_3-CH_2-$), 5.92 (1H, dd, $J_{6-7}$ = 4 Hz, $J_{7-NH}$ = 8 Hz, $C_7$-H), and 7.20 - 7.70 (5H, m, aromatic protons).

c) tert.Butyl 7β-(2-phenyl-2-diethylphosphoryloxyiminoacetamido)cephalosporanate.

The oxime prepared according to subsection b) (160 mg), triethylamine (42 mg), and diethyl phosphorochloridate (71 mg) were dissolved in tetrahydrofuran (15 ml) and the mixture was stirred at room temperature for 14 hours. After removal of the tetrahydrofuran by evaporation, the residual oil was extracted with ethyl acetate, washed with water, and dried over sodium sulphate. The ethyl acetate was evaporated off to yield an oil. By preparative TLC (silica gel, toluene/ethyl acetate = 1/1), there was obtained 80 mg of the compound (yield: 39%).

IR $\nu$ (max., KBr) $cm^{-1}$: 1785, 1730, 1680 (C=O), 1260 (P=O), and 1030 (P-O-C).

NMR (in acetone-$d_6$) $\delta$ : 1.34 (6H, t, J = 8 Hz, $-P(O)(OCH_2C\underline{H}_3)_2$), 1.54 (9H, s, $-C(CH_3)_3$), 2.00 (3H, s, $-COCH_3$), 3.45 (1H, d, J = 18 Hz, $C_2$-H), 3.75 (1H, d, J = 18 Hz, $C_2$-H), 4.20 (4H, m, $-P(O)(OC\underline{H}_2CH_3)_2$), 4.70 (1H, d, J = 12 Hz, $C_3-CH_2-$), 5.02 (1H, d, J = 12 Hz, $C_3-CH_2$), 5.22 (1H, d, $J_{6-7}$ = 5 Hz, $C_6$-H), 6.00 (1H, dd, $J_{6-7}$ = 5 Hz, $J_{7-NH}$ = 8 Hz, $C_7$-H), 7.40 - 7.80 (5H, m, aromatic protons), and 8.96 (1H, d, $J_{7-NH}$ = 8 Hz, NH).

d) 7β-(2-Phenyl-2-diethylphosphoryloxyiminoacetamido)cephalosporanic acid.

The ester prepared according to subsection c) (50 mg) was dissolved in trifluoroacetic acid (1.0 ml) and stirred for 2 hours at a

temperature below 5°C under nitrogen atmosphere. The trifluoroacetic acid was evaporated off to yield an oil, which was extracted with ethyl acetate and washed with water. The product was extracted from the ethyl acetate phase with an aqueous solution of sodium bicarbonate and the aqueous phase was acidified to a pH value of 2.0 with 1N hydrochloric acid. The precipitated oil was extracted with ethyl acetate, washed with brine, dried over sodium sulphate, and evaporated to give 42 mg of a yellowish powder (yield: 93%).

IR ν (max., $CHCl_3$) $cm^{-1}$: 1785, 1740, 1690 (C=O), 1240 (P=O), and 1040 (P-O-C).

NMR (in $CDCl_3$) δ : 1.38 (6H, m, $-P(O)(OCH_2C\underline{H}_3)_2$), 2.05 (3H, s, $-COCH_3$), 3.30 (1H, d, J = 20 Hz, $C_2$-H), 3.60 (1H, d, J = 20 Hz, $C_2$-H), 4.20 - 4.40 (4H, m, $-P(O)(OC\underline{H}_2CH_3)_2$), 4.80 (1H, d, J = 14 Hz, $C_3-CH_2-$), 5.00 (1H, d, $J_{6-7}$ = 4 Hz, $C_6$-H), 6.05 (1H, d, J = 14 Hz, $C_3-CH_2-$), 5.80 (1H, dd, $J_{6-7}$ = 4 Hz, $J_{7-NH}$ = 10 Hz, $C_7$-H), and 7.30 - 7.70 (5H, m, aromatic protons).

Example 15.

The following compounds can be prepared analogously to the process described in Example 1:

a) 7β-[2-(2-Aminothiazol-4-yl)-2-diisopropylphosphoryloxyimino-acetamido]cephalosporanic acid.

b) 7β-[2-(2-Aminothiazol-4-yl)-2-di(2-butyl)phosphoryloxyiminoacetamido]cephalosporanic acid.

c) 7β-[2-(2-Aminothiazol-4-yl)-2-bis(2-cyanoethyl)phosphoryloxyiminoacetamido]cephalosporanic acid.

d) 7β-[2-(2-Aminothiazol-4-yl)-2-(2-oxo-1,3,2-dioxaphosphorin-2-yloxyimino)acetamido]cephalosporanic acid.

e) 7β-[2-(2-Aminothiazol-4-yl)-2-dibenzylphosphoryloxyiminoacetamido]cephalosporanic acid.

f) 7β-[2-(2-Aminothiazol-4-yl)-2-bis(2,2,2-trifluoroethyl)phosphoryloxyiminoacetamido]cephalosporanic acid.

g) 7β-[2-(2-Aminothiazol-4-yl)-2-bis(N,N-dimethylamino)phosphoryloxyiminoacetamido]cephalosporanic acid.

h) 7β-[2-(2-Aminothiazol-4-yl)-2-bis(methylamino)phosphoryloxyiminoacetamido]cephalosporanic acid.

i) 7β-[2-(2-Aminothiazol-4-yl)-2-dipiperidinophosphoryloxyimino-acetamido]cephalosporanic acid.

j) 7β-[2-(2-Aminothiazol-4-yl)-2-(1,3-dimethyl-2-oxo-1,3,2-diazaphosphorin-2-yloxyimino)acetamido]cephalosporanic acid.

k) 7β-[2-(2-Aminothiazol-4-yl)-2-([ethyl]phenylphosphonyloxyimino)-acetamido]cephalosporanic acid.

l) 7β- [2-(2-Aminothiazol-4-yl)-2-( [morpholino]phenylphosphonyloxyimino)acetamido]cephalosporanic acid.

m) 7β- [2-(2-Aminothiazol-4-yl)-2-( [ethyl] pyrid-4-ylphosphonyloxyimino)-acetamido]cephalosporanic acid.

n) 7β- [2-(2-Aminothiazol-4-yl)-2-( [4-methoxybenzyl]methylphosphonyloxyimino)acetamido]cephalosporanic acid.

o) 7β- [2-(2-Aminothiazol-4-yl)-2-( [morpholino] methylphosphonyloxyimino)acetamido]cephalosporanic acid.

p) 7β-[2-(2-Aminothiazol-4-yl)-2-dimethylphosphoryloxyiminoacetamido] -3-carbamoyloxymethylceph-3-em-4-carboxylic acid.

q) 7β- [2-(2-Aminothiazol-4-yl)-2-dimethylphosphoryloxyiminoacetamido]-3-methylceph-3-em-4-carboxylic acid.

r) 7β- [2-(2-Aminothiazol-4-yl)-2-dimethylphosphoryloxyiminoacetamido]-3-(1-methyltetrazol-5-ylthiomethyl)ceph-3-em-4-carboxylic acid.

s) 7β- [2-(2-Aminothiazol-4-yl)-2-dimethylphosphoryloxyiminoacetamido]-3-(2-methyl-1,3,4-thiadiazol-5-ylthiomethyl) ceph-3-em-4-carboxylic acid.

t) 7β- [2-(2-Aminothiazol-4-yl)-2-dimethylphosphoryloxyiminoacetamido ]-3-methylthiomethylceph-3-em-4-carboxylic acid.

u) 7β- [2-(2-Aminothiazol-4-yl)-2-dimethylphosphoryloxyiminoacetamido]-3-(1- [2-(N,N-dimethylamino)ethyl]tetrazol-5-ylthiomethyl)-ceph-3-em-4-carboxylic acid.

v) 7β- [2-(2-Aminothiazol-4-yl)-2-dimethylphosphoryloxyiminoacetamido]-3-(1- [diethylphosphonylmethyl]tetrazol-5-ylthiomethyl)ceph-3-em-4-carboxylic acid.

x) 7β-[2-(2-Aminothiazol-4-yl)-2-diethylphosphoryloxyiminoacetamido]-3-(1-methyltetrazol-5-ylthiomethyl)ceph-3-em-4-carboxylic acid.

y) 7β-[2-(2-Aminothiazol-4-yl)-2-dimorpholinophosphoryloxyiminoacetamido]-3-(1-methyltetrazol-5-ylthiomethyl)ceph-3-em-4-carboxylic acid.

z) 7β-[2-(2-Aminothiazol-4-yl-2-([methyl] methylphosphonyloxyimino)acetamido]-3-(1-methyltetrazol-5-ylthiomethyl)ceph-3-em-4-carboxylic acid.

aa) 7β-[2-(4-Aminopyrimidin-2-yl)-2-diethylphosphoryloxyiminoacetamido]cephalosporanic acid.

ab) 7β-[2-(4-Aminophyrimidin-2-yl)-2-dimethylphosphoryloxyiminoacetamido]cephalosporanic acid.

ac) 7β-[2-(4-Aminopyrimidin-2-yl)-2-dianilinophosphoryloxyiminoacetamido]cephalosporanic acid.

ad) 7β-[2-(4-Aminopyrimidin-2-yl)-2-(2-oxo-1,3,2-dioxaphosphorin-2-yloxyimino)acetamido]cephalosporanic acid.

ae) 7β-[2-(4-Aminopyrimidin-2-yl)-2-bis(N,N-dimethylamino)phosphoryloxyiminoacetamido]cephalosporanic acid.

af) 7β-[2-(4-Aminopyrimidin-2-yl)-2-dipiperidinophosphoryloxyiminoacetamido]cephalosporanic acid.

ag) 7β-[2-(4-Aminopyrimidin-2-yl)-2-([methyl]methylphosphonyloxyimino)-acetamido]cephalosporanic acid.

ah) 7β-[2-(4-Aminopyrimidin-2-yl)-2-dimethylphosphoryloxyiminoacetamido]-3-(1-methyltetrazol-5-ylthiomethyl)ceph-3-em-4-carboxylic acid.

ai) 7β-[2-(2-Aminothiazol-4-yl)-2-morpholinomethylphosphoryloxyiminoacetamido]cephalosporanic acid.

aj) 7β-[2-(4-Aminopyrimidin-2-yl)-2-morpholinomethylphosphoryloxyiminoacetamido]cephalosporanic acid.

Pharmacological data:

The antibiotic activity in vitro of some compounds according to the invention and of some previously known compounds was tested by means of an agar serial dilution test. The following compounds were tested: 7β-[2-(2-Aminothiazol-4-yl)-2-diethylphosphoryloxyiminoacetamido] cephalosporanic acid (designated ES 643, 7β-[2-(2-amino-thiazol-4-yl)-2-dimethylphosphoryloxyiminoacetamido] cephalosporanic acid (designated ES 671), 7β-[2-(2-amino-thiazol-4-yl)-2-dimorpholinophosphoryloxyiminoacetamido] cephalosporanic acid (designated ES 656), 7β-[2-(2-amino-thiazol-4-yl)-2-methylmethylphosphonyloxyiminoacetamido] cephalosporanic acid (designated ES 682), 7β-[2-(2-amino-thiazol-4-yl)-2-hydroxyiminoacetamido] cephalosporanic acid (designated A), cefuroxime (designated B), and cephalotin (designated C). MIC designates minimum inhibitory concentration.

| test organism | MIC value in $\mu g/ml$ ES 643 | ES 671 | ES 656 | ES 682 | A | B | C |
|---|---|---|---|---|---|---|---|
| S. aureus 209P | 3.1 | 3.1 | 0.2 | 0.4 | 1.6 | 1.6 | <0.1 |
| S. aureus 13709 | 3.1 | 1.6 | 0.2 | 0.8 | 1.6 | 0.8 | 0.4 |
| S. aureus 3349* | 1.6 | 1.6 | 0.4 | 0.4 | 1.6 | 0.8 | 0.4 |
| S. aureus 3356* | 1.6 | 1.6 | 0.2 | 0.4 | 0.8 | 0.8 | <0.1 |
| S. albus 3348 | 0.8 | 0.8 | 0.2 | 0.2 | 0.8 | 0.2 | <0.1 |
| S. albus 3578 | 1.6 | 0.8 | 0.2 | 0.2 | 0.8 | 0.2 | <0.1 |
| S. albus 12228 | 1.6 | 0.4 | 0.2 | 0.2 | 0.8 | 0.4 | 0.2 |
| S. pyogenes 12334 | <0.1 | <0.1 | <0.1 | <0.1 | <0.1 | <0.1 | <0.1 |
| S. pyogenes 12384 | <0.1 | <0.1 | <0.1 | <0.1 | <0.1 | <0.1 | <0.1 |
| E. coli 01 | 0.2 | 0.2 | <0.1 | <0.1 | <0.1 | 3.1 | 6.2 |
| E. coli 04 | 0.8 | 0.4 | 0.2 | <0.1 | 0.2 | 6.2 | 12.5 |
| K. edwardsii X1 | 0.8 | 0.2 | 1.6 | <0.1 | 0.2 | 6.2 | 0.2 |
| K. pneumoniae K8 | 1.6 | 0.4 | 0.4 | 0.2 | 0.4 | 12.5 | 50 |
| P. mirabilis 682 | <0.1 | <0.1 | <0.1 | <0.1 | <0.1 | 3.1 | 6.2 |
| P. mirabilis 2255 | <0.1 | 0.1 | 0.2 | <0.1 | <0.1 | 1.6 | 3.1 |
| P. mirabilis 2311 | <0.1 | <0.1 | 0.2 | <0.1 | <0.1 | 0.8 | 12.5 |
| P. morganii 18 | 1.6 | <0.1 | 1.6 | <0.1 | 0.2 | 25 | >100 |
| P. morganii 69 | 0.2 | 0.4 | 3.1 | <0.1 | 0.2 | 25 | >100 |
| P. vulgaris 5209 | 0.8 | 0.2 | 50 | 0.1 | 6.2 | >100 | >100 |
| P. vulgaris 16868 | 0.2 | <0.1 | 12.5 | <0.1 | 1.6 | >100 | >100 |
| P. vulgaris 17028 | 0.2 | <0.1 | 25 | <0.1 | 0.8 | >100 | >100 |

* = penicillin-resistant strain

CLAIMS

1. Cephalosporanic acid derivatives of the general formula

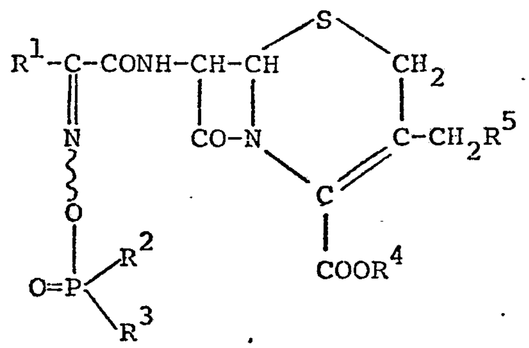

(I)

wherein $R^1$ represents an optionally substituted 5 or 6 membered aromatic or hetero aromatic group, $R^2$ and $R^3$ are the same or different and each represents lower alkyl, substituted lower alkyl, aryl, substituted aryl, aralkyl, substituted aralkyl, a 5 or 6 membered heterocyclic group containing one or two hetero atoms selected from the group consisting of nitrogen, oxygen, and sulphur, or a group of the general formula $-OR^6$, wherein $R^6$ represents hydrogen, a salt forming cation, lower alkyl, substituted lower alkyl, aryl, substituted aryl, aralkyl, substituted aralkyl, or a 5 or 6 membered heterocyclic group containing one or two hetero atoms selected from the group consisting of nitrogen, oxygen, and sulphur, or $R^2$ and/or $R^3$ (each) represent(s) a group of the general formula $-NR^7R^8$, wherein $R^7$ and $R^8$ are the same or different and each represents hydrogen, lower alkyl, substituted lower alkyl, a 5 or 6 membered cycloalkyl group, aryl, or substituted aryl, or $R^7$ and $R^8$ together with the adjacent nitrogen atom form a 5 or 6 membered heterocyclic ring which may contain a further hetero atom selected from the group consisting of nitrogen, oxygen, and sulphur, or $R^2$ and $R^3$ together with the adjacent phosphorus atom form a 5 or 6 membered heterocyclic ring which may contain one or more further hetero atom(s) selected from the group consisting of nitrogen, oxygen, and sulphur, and which may be substituted, $R^4$ represents hydrogen, a salt forming cation, or an ester forming group, and $R^5$ represents hydrogen, acyloxy, carba-

moyloxy, substituted or unsubstituted pyridyl, or a group of the general formula $-SR^9$, wherein $R^9$ represents lower alkyl or a 5 or 6 membered nitrogen containing heterocyclic group, which may contain further hetero atoms, e.g. sulphur and oxygen, and which may be substituted, whereby the wave line designates that the group

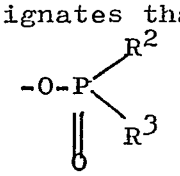

is "syn" or "anti" or mixtures thereof, and salts and hydrates thereof, preferably physiologically acceptable salts thereof.

2. A derivative according to Claim 1, wherein $R^1$ represents a group of the general formula

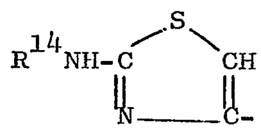

, wherein $R^{14}$ represents hydrogen or a group easily removable by hydrolysis or hydrogenolysis, preferably synisomers, and salts and hydrates thereof, preferably physiologically acceptable salts thereof.

3. A derivative according to Claim 1, wherein $R^1$ represents phenyl, furyl, thiazolyl, or pyrimidinyl each of which may be substituted by amino, $R^2$ and $R^3$ are the same or different and each represents hydroxy, phenoxy, lower alkyl, lower alkoxy which may be substituted by one, two, or three halogen atom(s), or a group of the formula $-NR^7R^8$, wherein $R^7$ and $R^8$ are the same or different and each represents hydrogen, lower alkyl, or phenyl, or wherein $R^7$ and $R^8$ together with the adjacent nitrogen atom from a 6 membered heterocyclic, saturated ring which may contain a further hetero atom, i.e. an oxygen atom, $R^4$ represents hydrogen or a salt forming ion, and $R^5$ represents acyloxy containing not more than 7 carbon atoms, 1-methyltetrazol-5-ylthio, or 2-methyl-1,3,4- thiadiazol-5-ylthio, preferably syn-isomers, and salts and hydrates thereof, preferably physiologically acceptable salts thereof.

4. A derivative according to Claim 1, wherein $R^1$ represents phenyl, furyl, thiazolyl, or pyrimidinyl each of which may be substituted by amino, $R^2$ and $R^3$ are the same or different and each represents hydroxy, lower alkyl, lower alkoxy, 2,2,2-trichloroethyoxy, phenoxy, N,N-diethylamino, anilino, or morpholino, $R^4$ represents hydrogen or a salt forming ion, and $R^5$ represents acyloxy containing not more than 7 carbon atoms, 1-methyltetrazol-5-ylthio, or 2-methyl-1,3,4- thiadiazol-5-ylthio, preferably syn-isomers, and salts and hydrates thereof, preferably physiologically acceptable salts thereof.

5. A derivative according to Claim 1, wherein $R^1$ represents phenyl, furyl, thiazolyl, or pyrimidinyl which may be substituted by amino, $R^2$ and $R^3$ each represents hydroxy, lower alkyl, lower alkoxy, or morpholino, $R^4$ represents hydrogen or sodium, and $R^5$ represents acetoxy, preferably syn-isomers, and salts and hydrates thereof, preferably physiologically acceptable salts thereof.

6. A derivative according to Claim 1, wherein the group of the formula $-O-(O=)P(R^2)(R^3)$ is in the syn-position.

7. A derivative according to Claim 2, wherein $R^2$ and $R^3$ are the same or different and each represents hydroxy, phenoxy, lower alkyl, lower alkoxy which may be substituted by one, two, or three halogen atom(s), or a group of the formula $-NR^7R^8$, wherein $R^7$ and $R^8$ are the same or different and each represents hydrogen, lower alkyl, or phenyl, or wherein $R^7$ and $R^8$ together with the adjacent nitrogen atom form a 6 membered heterocyclic, saturated ring which may contain a further hetero atom, i.e. an oxygen atom, $R^4$ represents hydrogen or a salt forming ion, and $R^5$ represents

acyloxy containing not more than 7 carbon atoms, 1-methyltetrazol-5-ylthio, or 2-methyl-1,3,4-thiadiazol-5-ylthio, preferably syn-isomers, and salts and hydrates thereof, preferably physiologically acceptable salts thereof.

8. A derivative according to Claim 1, wherein $R^1$ represents phenyl, furyl, thiazolyl, or pyrimidinyl, each of which may be substituted with amino, $R^2$ and $R^3$ each represents lower alkyl or lower alkoxy

which may be substituted with up to 3 halogen atoms; hydroxy;phenoxy; amino which may be substituted with one or two lower alkyl groups or with phenyl; or morpholino, $R^4$ represents hydrogen or a salt forming ion, and $R^5$ represents acyloxy containing not more than 7 carbon atoms, 1-methyltetrazol-5-ylthio, or 2-methyl-1,3,4-thiadiazol-5-ylthio, preferably syn-isomers, and salts and hydrates thereof, preferably physiologically acceptable salts thereof.

9. 7β-[2-(2-Aminothiazol-4-yl)-2-diethylphosphoryloxyiminoacetamido]cephalosporanic acid, 7β-[2-(2-aminothiazol-4-yl)-2-dimethylphosphoryloxyiminoacetamido]cephalosporanic acid, 7β-[2-(2-aminothiazol-4-yl)-2-bis(2,2,2-trichloroethyl)phosphoryloxyiminoacetamido]cephalosporanic acid, 7β-[2-(2-aminothiazol-4-yl)-2-dimorpholinophosphoryloxyiminoacetamido]cephalosporanic acid, 7β-[2-(2-aminothiazol-4-yl)-2-dianilinophosphoryloxyiminoacetamido]cephalosporanic acid, 7β-[2-(2-aminothiazol-4-yl)-2-diphenylphosphoryloxyiminoacetamido]cephalosporanic acid, 7β-[2-(2-aminothiazol-4-yl)-2-([methyl]methylphosphonyloxyimino)acetamido]cephalosporanic acid, 7β-[2-(2-aminothiazol-4-yl)-2-methylphosphoryloxyiminoacetamido]cephalosporanic acid, 7β-[2-(2-aminothiazol-4-yl)-2-methylethylphosphonyloxyiminoacetamido]cephalosporanic acid, 7β-[2-(2-aminothiazol-4-yl)-2-phenylethylphosphonyloxyiminoacetamido]cephalosporanic acid, 7β-[2-(2-aminothiazol-4-yl)-2-(N,N-diethylamino)methylphosphonyloxyiminoacetamido]cephalosporanic acid, 7β-[2-(2-aminothiazol-4-yl)-2-ethylmethylphosphonyloxyiminoacetamido]cephalosporanic acid, 7β-[2-(fur-2-yl)-2-diethylphosphoryloxyiminoacetamido)cephalosporanic acid, 7β-(2-phenyl-2-diethylphosphoryloxyiminoacetamido)cephalosporanic acid, 7β-[2-(2-aminothiazol-4-yl)-2-morpholinomethylphosphoryloxyiminoacetamidocephalosporanic acid, or 7β-[2-(4-aminopyrimidin-2-yl)-2-morpholinomethylphosphoryloxyiminoacetamido]cephalosporanic acid in syn-form and salt and hydrates thereof, preferably sodium salts.

10. A process for the preparation of compounds of the general formula I in accordance with Claim 1 and salts and hydrates thereof, characterized by

a) reacting a compound of the general formula

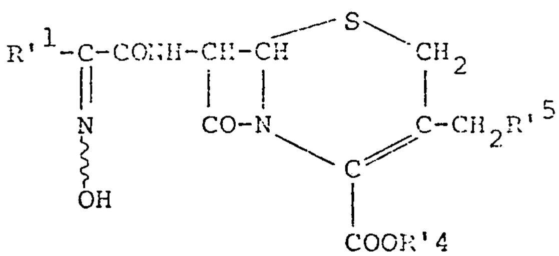

(II)

wherein $R'^1$ and $R'^5$, respectively, each has the same meaning as $R^1$ and $R^5$, respectively, or represents groups convertible thereto, and $R'^4$ represents a protecting group easily removable by hydrolysis or hydrogenolysis, with a compound of the general formula

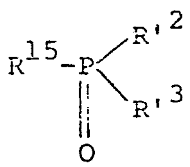

(III)

wherein $R'^2$ and $R'^3$, respectively, each has the same meaning as $R^2$ and $R^3$, respectively, or represents groups convertible thereto, and $R^{15}$ represents a leaving group commonly used as an activating group on a phosphorylating agent, or

b) reacting a compound of the general formula

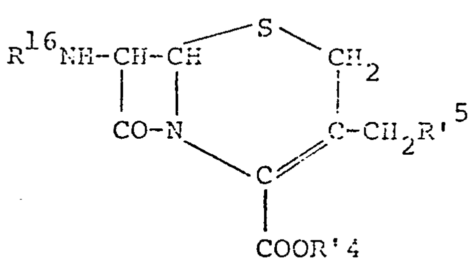

(IV)

wherein $R'^4$ and $R'^5$ each is as defined above, and $R^{16}$ represents hydrogen or a leaving group commonly connected to an amino group which is to be acylated, or a salt thereof with a compound of the general formula

BAD ORIGINAL

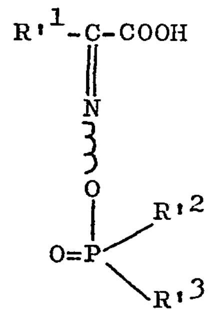

wherein $R'^1$, $R'^2$, and $R'^3$, each is as defined above, or an activated derivative thereof, and, if necessary, subsequent to process variant a) or b) converting one or more of the groups $R'^1$, $R'^2$, $R'^3$, $R'^4$ or $R'^5$, respectively, into the corresponding groups $R^1$, $R^2$, $R^3$, $R^4$ or $R^5$, respectively, and/or, if desired, converting one or more of the groups $R^1$, $R^2$, $R^3$, $R^4$ or $R^5$, respectively, into other groups within the definitions of $R^1$, $R^2$, $R^3$, $R^4$ or $R^5$, respectively, and, if desired, converting a compound of formula I into a salt or into a hydrate thereof, or, if desired, converting a salt or a hydrate of a compound of the formula I into a compound of the formula I.

11. A pharmaceutical composition which comprises an effective amount of at least one derivative in accordance with any one of Claims 1-9, in association with a suitable physiologically acceptable carrier or excipient.

12. The use of a derivative in accordance with any one of Claims 1-9 as a therapeutic agent against a micro-organism.